# FASCICULE DE BREVET EUROPEEN

(11) **EP 2 909 171 B1**
(45) Date de publication et mention de la délivrance du brevet: **28.09.2016**
(21) Numéro de dépôt: 13779187.7
(22) Date de dépôt: 14.10.2013
(51) Int. Cl.: C07D 207/333, A61K 31/40, A61P 35/00

(54) **COMPOSÉS 3,4-BIS(CATÉCHOL)PYRROLE N-SUBSTITUÉS, LEUR PRÉPARATION ET UTILISATION DANS LE TRAITEMENT DU CANCER**
N-SUBSTITUIERTE 3,4-BIS(CATECHOL)PYRROL-VERBINDUNGEN, DEREN HERSTELLUNG UND VERWENDUNG IN DER BEHANDLUNG VON KREBS
N-SUBSTITUTED 3,4-BIS(CATECHOL)PYRROLE COMPOUNDS, THEIR PREPARATION AND USE IN THE TREATMENT OF CANCER

(30) Priorité: 16.10.2012 FR 1259868
(43) Date de publication de la demande: 26.08.2015
(73) Titulaire: Centre National de la Recherche Scientifique (CNRS), 75016 Paris (FR)
(72) Inventeur: COLLIN, Pascal, F-78530 Buc (FR); EGOROV, Maxim, F-44340 Bouguenais (FR); DELPECH, Bernard, F-92140 Clamart (FR); BAKALA, Joanna, F-75012 Paris (FR); ACHAB, Maria, F-91190 Gif s/Yvette (FR); BIGNON, Jérôme, F-91530 Le Val Saint Germain (FR); THOISON, Odile, F-91120 Palaiseau (FR); BENECHIE, Michel, F-91190 Gif s/Yvette (FR)
(74) Mandataire: Regimbeau
(86) Numéro de dépôt international: PCT/EP2013/071445
(87) Numéro de publication internationale: WO 2014/060366

(56) Documents cités:
- WO-A1-00/20411
- WO-A1-2008/064317
- YOEL KASHMAN ET AL: "Halitulin, a new cytotoxic alkaloid from the marine sponge Haliclona tulearensis", TETRAHEDRON LETTERS, vol. 40, no. 5, 1999, pages 997-1000, XP004151502, ISSN: 0040-4039, DOI: 10.1016/S0040-4039(98)02467-8
- DAVID E. WILLIAMS ET AL: "Motuporamines, anti-invasion and anti-angiogenic alkaloids from the marine sponge Xestospongia exigua (Kirkpatrick): isolation, structure elucidation, analogue synthesis, and conformational analysis", JOURNAL OF ORGANIC CHEMISTRY, vol. 67, no. 1, 2002, pages 245-258, XP002195935, ISSN: 0022-3263, DOI: 10.1021/JO016101C cité dans la demande

## Description

La présente invention est relative à de nouveaux composés 3,4-bis(catéchol)pyrrole-N-substitués, leur préparation et utilisation dans le traitement du cancer.

Les cellules tumorales ont pour origine des modifications chromosomiques innée ou acquise résultant de translocation, amplification génique, insertion rétrovirale, mutation, délétion génétique, ainsi que de désordres épigénétiques. Ces anomalies touchent de nombreux gènes impliqués dans le contrôle du cycle cellulaire, de l'apoptose, de la croissance et de la survie, ainsi que les gènes de réparation, stabilisation de l'ADN et de détoxification.

La grande majorité de la chimiothérapie anticancéreuse traditionnelle repose principalement sur l'utilisation de drogues dont le mode d'action passe par le blocage de la multiplication cellulaire, soit en phase S du cycle cellulaire en s'attaquant à l'ADN de la cellule ou en phase G2/M en s'attaquant aux microtubules du fuseau mitotique nécessaire à la division cellulaire. D'autres agents anticancéreux visent à déclencher une mort cellulaire programmée par apoptose, en induisant un stress oxydatif. L'inconvénient de ces approches est la relative non-spécificité des médicaments qui ne peuvent épargner les cellules saines. Il s'ensuit des effets secondaires multiples. Plus récemment, ont été développés de nouveaux axes thérapeutiques n'utilisant plus une action cytotoxique, comme moyen de guérir, mais plutôt l'utilisation d'un frein pour ralentir ou arrêter le développement tumoral. Cette chimiothérapie ciblée vise les mécanismes intimes de la prolifération cellulaire en bloquant la multiplication des cellules, par inhibition des voies de transmission des signaux de facteurs de croissance, ou par inhibition de l'angiogénèse.

Cependant, avec ces nouveaux traitements anticancéreux ciblés, de nouvelles chimiorésistances sont apparues. Elles diffèrent des mécanismes connus de chimiorésistance liés principalement à l'expression de système membranaire d'expulsion de drogues : protéine ABC (ATP-Binding Cassette) dont fait partie le système MDR1 (Multi Drug Resistance-1). Dans ce cas, les cellules cancéreuses développent une chimiorésistance en stimulant la voie de survie, associée à l'inhibition des voies pro-apoptotiques. Cette voie de survie est activée par les récepteurs à activité tyrosine kinase (EGFR, PDGFR, IGFR), eux même activés par les facteurs de croissance. Elle est constituée par les protéines PI3K (Phosphatidyl inositol 3 OH kinase), PDK (phosphoinositide dépendent tyrosine kinase), Akt (serine/thréonine protein kinase PKB), PTEN (phosphatase and tensin homologue deleted on chromosome 10), FOXO (forkhead box O), mTOR (mamalian target of rapamycin), associées aux oncogènes Src et Ras. D'une manière générale, la voie de survie a un effet protecteur contre l'apoptose et peut induire l'autophagie.

Le lien entre la voie de survie et la cancérogénèse est maintenant bien établi. En effet, des mutations dans les gènes codant pour la voie de survie sont présentes dans plus de 30% des cancers dont 70% des cancers du sein : mutation activatrice des gènes PIK3CA, PIK3CB, AKT1, AKT2, perte de fonction de PTEN, et surexpression des oncogènes src et Ras, régulant cette voie.

Ainsi, depuis quelques années, se sont développés des composés inhibiteurs de cette voie de survie pour lutter efficacement contre les cellules cancéreuses, qui en association avec d'autres drogues, bloquent la transmission des signaux des facteurs de croissance.

Les essais cliniques récents ont révélé que l'inhibition de la voie de survie bloquait la tumorigénèse. Ainsi, l'utilisation d'inhibiteur de la voie de survie comme traitement anticancéreux peut être considéré comme une nouvelle arme contre le cancer.

L'un des principaux objectifs de la pharmacologie des médicaments anticancéreux est donc la recherche permanente de nouvelles drogues susceptibles de manifester une meilleure efficacité thérapeutique, à travers des cibles moléculaires spécifiques, associée à l'absence de chimiorésistance ainsi que l'absence de toxicité sur les cellules saines.

Ces critères fondamentaux ont été retenus pour la sélection d'un nouveau médicament antitumoral.

La présente invention concerne les nouveaux composés 3,4-bis(catéchol)pyrrole-N-substitués de formule générale (I) ci-après, leur préparation ainsi que leur action antimitotique et leur mode d'action vis-à-vis de cellules cancéreuses. 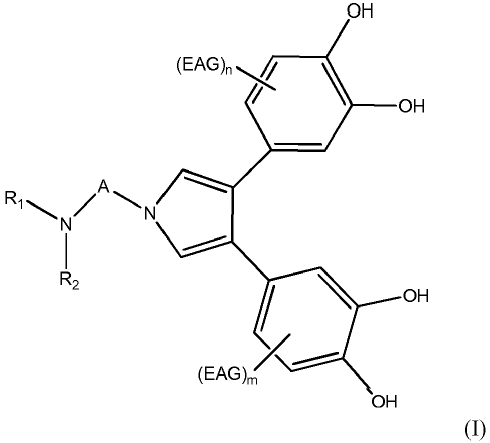 dans laquelle :
- m est un entier de 0 à 3, de préférence de 0 à 2 ;
- n est un entier de 0 à 3, de préférence de 0 à 2;
- m + n ≥ 1
- EAG représente un groupe électroattracteur indépendamment choisi parmi un atome d'halogène, un groupe NO₂, CF₃, CCl₃, CN, CO₂H, (C=O)NR₂, CH=NR, (C=S)OR, (C=O)SR, CS₂R, SO₂R, SO₂NR₂, SO₃R, P(O)(OR)₂, P(O)(R)₂, B(OR)₂, où R est un radical (C₁ - C₆) alkyle, un groupe phényle ou un atome d'hydrogène;
- A représente une chaîne hydrocarbonée, saturée ou insaturée, linéaire ou ramifiée, comprenant de 1 à 10 atomes de carbone ; et
- R₁ et R₂ représentent chacun, indépendamment l'un de l'autre, un atome d'hydrogène, un groupe CO-(C₁ - C₆)-alkyle, (C₁ - C₆) alkyle, phényle ou phényle-(C₁ - C₆)-alkyle, ou R₁ et R₂ forment ensemble, avec l'atome d'azote qui les porte, un hétérocycle à 5 à 15 chaînons, éventuellement substitué par un groupe (C₁ - C₆) alkyle ;
y compris ses stéréoisomères et leurs mélanges, ou un sel d'acide pharmaceutiquement acceptable de celui-ci.

La présente invention concerne en particulier le composé de formule (Ia) ainsi que son utilisation en tant que médicament pour le traitement du cancer.

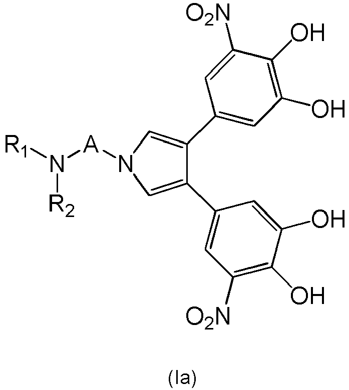

En particulier, les radicaux R₁ et R₂ forment ensemble, avec l'atome d'azote qui les porte, un hétérocycle qui est saturé et qui ne comprend pas d'autre hétéroatome que l'atome d'azote qui porte R₁ et R₂. Plus particulièrement R₁ et R₂ forment ensemble, avec l'atome d'azote qui les porte, un hétérocycle de 10 à 15 chaînons, de préférence à 13 chaînons.

En particulier, la chaîne A est une chaîne hydrocarbonée linéaire saturée comprenant 3 à 6 atomes de carbone, de préférence 3 atomes de carbone.

La présente invention concerne en particulier le composé de formule (1) ou un sel d'acide pharmaceutiquement acceptable de celui-ci 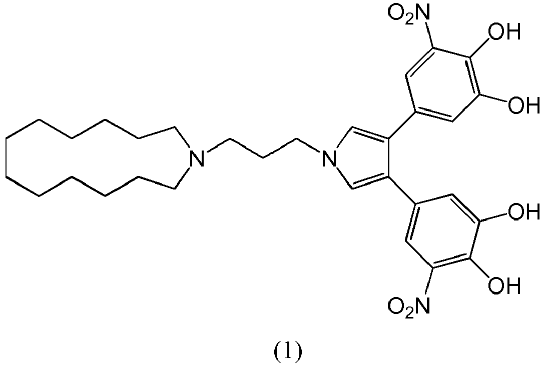 ainsi que son utilisation en tant que médicament dans le traitement du cancer.

On soulignera qu'*in vitro*, le composé (*1*), utilisé aux concentrations encadrant son IC50, ne manifeste aucune cytotoxicité vis-à-vis des cellules humaines normales.

En particulier, le sel d'acide pharmaceutiquement acceptable préféré de la formule générale (I), plus particulièrement le composé (Ia) et préférentiellement le composé (1), est un chlorhydrate.

La présente invention s'étend également à une composition pharmaceutique comprenant au moins un composé de formule générale (I), plus particulièrement le composé (Ia) et préférentiellement le composé (1), avec au moins un excipient pharmaceutiquement acceptable.

La présente invention concerne l'utilisation des composés (I), plus particulièrement (Ia) et (1), en tant que médicament dans le traitement du cancer, en particulier le cancer du poumon, du sein, du foie, de l'estomac, du côlon, de l'anus, de l'oesophage, du larynx, du nasopharynx, du pancréas, de la prostate, du rein, de la vessie, du duodénum, de l'endomètre, de la plèvre, de la peau, du testicule, de l'ovaire, de l'utérus, du cerveau, des os, de la bouche, de l'oeil, ou les cancers hématopoïétiques tels que les leucémies, les leucémies myéloïdes, les lymphomes, le cancer de la moelle osseuse ou encore les tumeurs d'origine neuroectodermique tels que les glioblastomes.

L'invention porte également sur un procédé de préparation d'un composé de formule (I), plus particulièrement (Ia) et (1) comprenant :
a) la réaction entre une amine de formule (II) suivante, un halogénure de formule (III) suivante, et un aldéhyde de formule (IV) suivante, 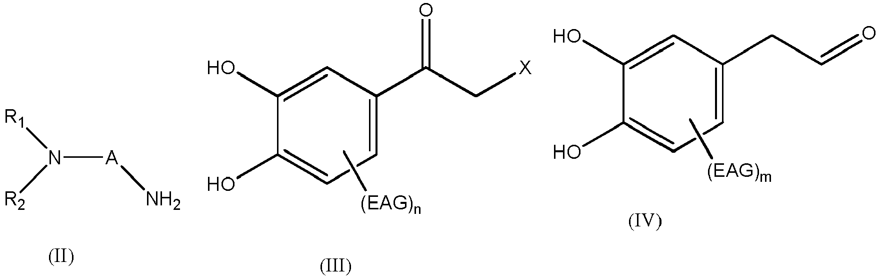 dans lesquelles n et m, EAG, R₁, R₂ et A sont tels que définis précédemment à propos de la formule générale (I), et X représente un atome d'halogène, pour donner un composé de formule (I).
b) éventuellement la formation d'un sel pharmaceutiquement acceptable à partir du composé de formule (I) obtenu à l'étape a) précédente.

Dans ce procédé de préparation l'halogénure (III) et l'amine (II) sont d'abord mis à réagir ensemble avant d'ajouter l'aldéhyde (IV). De préférence, le radical X de l'halogénure (III) est un atome de chlore, de brome ou d'iode.

### RESULTATS

### Synthèse chimique

Le composé 5,5'-(1-(3-(azacyclotridécan-1-yl)propyl)-1*H*-pyrrole-3,4-diyl)bis(3-nitrobenzène-1,2-diol) (*1*) a été préparé via la condensation de l'halogénure de phénacyle 3 avec l'amine primaire *2* et le phénylacétaldéhyde 4, représentés ci-après : 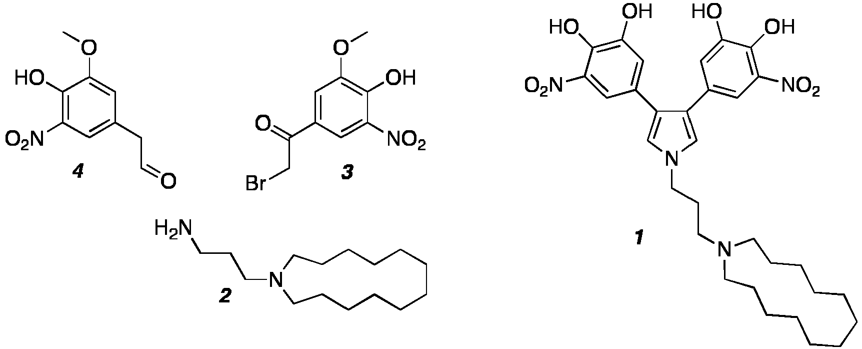 Le *N*-(3-aminopropyl)azacyclotridécane (*2*) ((*a*) G. Koren-Goldshlager, Y. Kashman, M. Schleyer, J. Nat. Prod., 1998, 61, 282-284; (*b*) D. E. Williams, K. S. Craig, B. Patrick, L. M. McHardy, R. van Soest, M. Roberge, R. J. Andersen, J. Org. Chem., 2002, 67, 245-258) a été obtenu à partir du laurolactame commercial selon le schéma 1 ci-après :

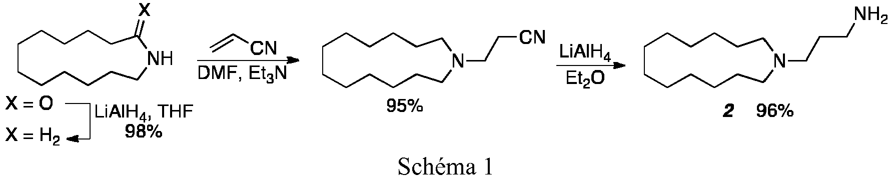

Les composés 3 (J. Borgulya, H. Bruderer, K. Bernauer, G. Zürcher, M. Da Prada, Helv. Chim. Acta, 1989, 72, 952-968) et 4 ont été préparés selon le schéma 2 ci-après :

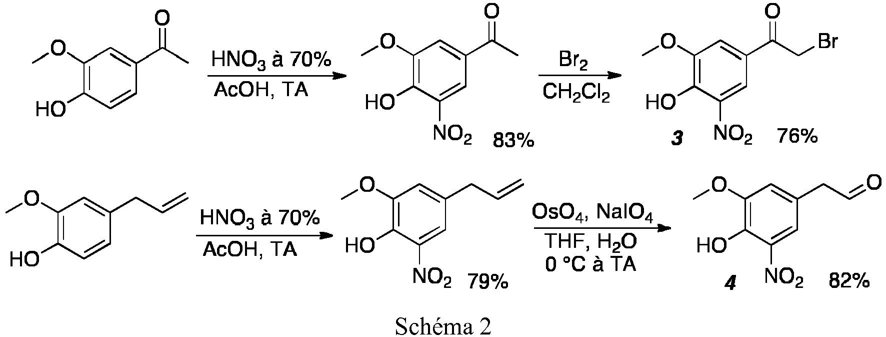

Une méthode de formation monotope a été mise au point pour la synthèse du pyrrole 5 en partant de l'amine 2, de l'halogénure 3 et du phénylacétaldéhyde 4 (Schéma 3).

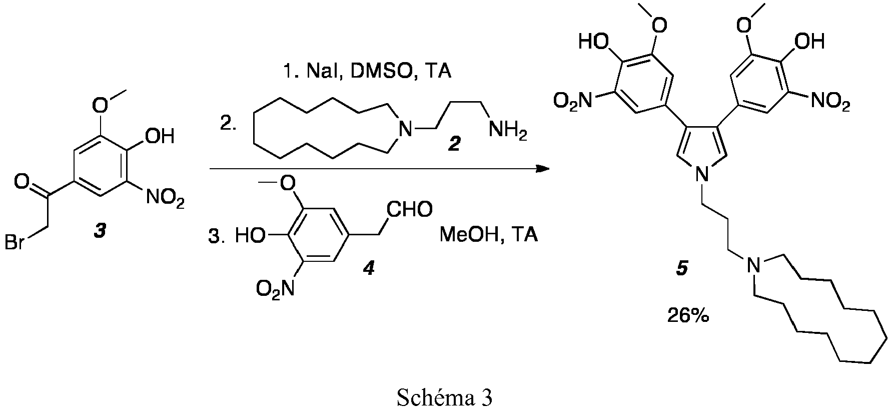

Par traitement du composé 5 avec une solution de BBr₃ dans le dichlorométhane, le produit déméthylé (1) a été obtenu (Schéma 4).

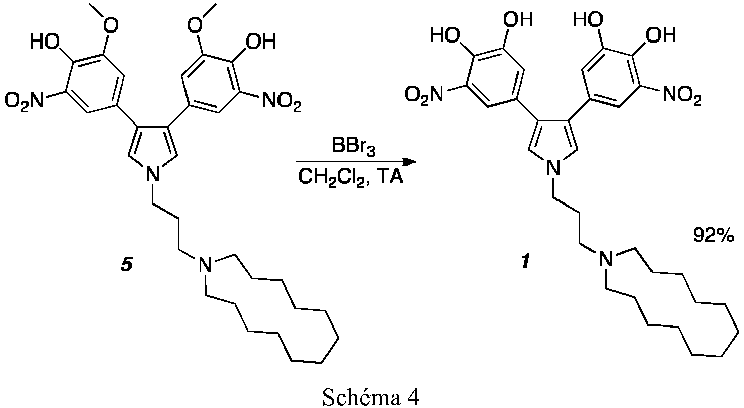

Le chlorhydrate correspondant a été obtenu par traitement avec une solution de HCl dans le méthanol.

### Partie expérimentale

### Préparation de la 3-(azacyclotridécan-1-yl)propan-1-amine (2)

Azacyclotridécane ((*a*) J. E. Baldwin, H. R. Vollmer, V. Lee, Tetrahedron Lett., 1999, 40, 5401-5404; (*b*) M. H. Weston, K. Nakajima, T. G. Back, J. Org. Chem., 2008, 73, 4630-4637).

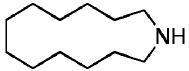

A une solution d'azacyclotridécan-2-one (4,3 g; 21,8 mmol) dans le THF (100 mL) à TA sous argon, on ajoute graduellement LiAlH₄ (1,5 g; 39,5 mmol) puis le mélange réactionnel est chauffé à reflux pendant 16 h. Une solution saturée de Na₂SO₄ est ensuite ajoutée graduellement à 0 °C pour neutraliser l'excès de LiAlH₄, puis on ajoute un mélange éther/pentane 1:1 (200 mL) et un excès de K₂CO₃ solide pour absorber toute la phase aqueuse. La phase organique est ensuite décantée et le précipité est lavé trois fois avec un mélange éther/pentane 1:1. Le solvant est éliminé sous pression réduite pour donner l'azacyclotridécane (3,9 g; 98%) sous forme d'huile incolore. RMN ¹H (CDCl₃; 300 MHz) *δ* (ppm) 2,62 (4 H; t; *J =* 5,1 Hz); 1,57-1,25 (20 H; m). RMN ¹³C (CDCl₃, 75 MHz) *δ* (ppm) 47,9; 27,9; 26,5; 26,0; 25,4; 24,6. 3-(Azacyclotridécan-1-yl)propanenitrile

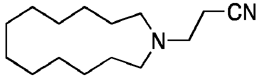

A une solution d'azacyclotridécane (3,8 g; 20,7 mmol) et de triéthylamine (50 µL; 0,35 mmol) dans le DMF (15 mL) sous argon, on ajoute de l'acrylonitrile (7,7 mL; 115 mmol) et le mélange réactionnel est agité pendant 16 h à 70 °C. Le mélange réactionnel est extrait avec de l'éther, la phase organique est lavée avec de la saumure, séchée sur Na₂SO₄, et évaporée sous pression réduite pour donner le 3-(azacyclotridécan-1-yl)propanenitrile sous forme d'huile incolore (4,64 g; 95%). RMN ¹H (CDCl₃; 300 MHz) *δ* (ppm) 2,76 (2 H; t; *J* = 7,2 Hz); 2,50-2,35 (6 H; m); 1,55-1,28 (20 H; m). RMN ¹³C (CDCl₃; 75 MHz) *δ* (ppm) 53,7; 49,7; 26,5; 26,2; 25,23; 25,18; 15,7.

3-(Azacyclotridécan-1-yl)propan-1-amine (2) ((*a*) G. Koren-Goldshlager, Y. Kashman, M. Schleter, J. Nat. Prod., 1998, 61, 282-284; (*b*) D. E. Williams, K. S. Craig, B. Patrick, L. M. McHardy, R. van Soest, M. Roberge, R. J. Andersen, J. Org. Chem., 2002, 67, 245-258).

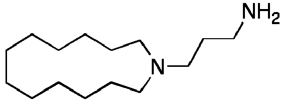

A une solution de 3-(azacyclotridecan-1-yl)propanenitrile (0,5 g; 2,1 mmol) dans l'éther (15 mL) à 0 °C sous argon, on ajoute graduellement LiAlH₄ (0,3 g; 7,9 mmol) et le mélange réactionnel est agité à 33 °C pendant 15 min. Une solution saturée de Na₂SO₄ est ensuite ajoutée graduellement à 0 °C pour neutraliser l'excès de LiAlH₄, puis du K₂CO₃ solide est ajouté pour absorber toute la phase aqueuse. La phase organique est décantée et le précipité est lavé trois fois avec de l'éther. Le solvant est éliminé sous pression réduite pour donner 2 (0,49 g, 96%) sous forme d'huile incolore. RMN ¹H (CDCl₃; 500 MHz) *δ* (ppm) 2,74 (2 H; t; *J* = 7,0 Hz); 2,40-2,28 (6 H; m); 1,57 (2 H; quint; *J* = 7,0 Hz); 1,44-1,32 (22 H; m). RMN ¹³C (CDCl₃; 125 MHz) *δ* (ppm) 54,4; 52,8; 41,2; 31,7; 26,7; 26,5; 26,3; 25,8; 25,6.

### Préparation de la 2-bromo-1-(4-hydroxy-3-méthoxy-5-nitrophényl)éthanone (3)

1-(4-Hydroxy-3-méthoxy-5-nitrophényl)éthanone ((*a*) L. E. Kiss, H. S. Ferreira, L. Torrão, M. J. Bonifácio, P. N. Palma, P. Soares-da-Silva, D. A. Learnmonth, J. Med. Chem., 2010, 53, 3396-3411; (*b*) X. Lu, S. Wan, J. Jiang, X. Jiang, W. Yang, P. Yu, L. Xu, Z. Zhang, G. Zhang, L. Shan, Y. Wang, Eur. J. Med. Chem., 2011, 46, 2691-2698).

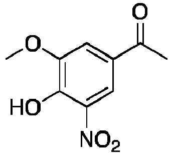

A une solution de 4'-hydroxy-3'-méthoxyacétophénone (10 g; 60,2 mmol) dans l'acide acétique (140 mL) à TA, on ajoute HNO₃ à 70% (4,2 mL; 66,3 mmol). Le mélange réactionnel est agité pendant 30 min et les cristaux jaunes sont filtrés, lavés avec de l'éther et séchés pour donner la 1-(4-hydroxy-3-méthoxy-5-nitrophényl)éthanone (10,5 g; 83%), pf 158-159 °C (lit. 159-161 °C, éthanol, X. Lu, S. Wan, J. Jiang, X. Jiang, W. Yang, P. Yu, L. Xu, Z. Zhang, G. Zhang, L. Shan, Y. Wang, Eur. J. Med. Chem., 2011, 46, 2691-2698). RMN ¹H (CDCl₃; 300 MHz) *δ* (ppm) 11,09 (1 H; s), 8,30 (1 H; d; *J* = 1,5 Hz); 7,75 (1 H; d; *J =* 1,5 Hz); 4,01 (3 H; s); 2,62 (3 H; s). RMN ¹³C (CDCl₃, 75 MHz) *δ* (ppm) 194,9; 150,4; 150,1; 133,0; 128,3; 117,7; 115,3; 56,9; 26,0. IR (neat) *v*ₘₐₓ cm⁻¹ 3228; 3091; 2988; 2925; 1676; 1612; 1535; 1413; 1378; 1350; 1329; 1218; 1136; 1047; 869; 735; 709; SMHR (ES) (*m*/*z*) [M-H]⁻ calculé pour C₉H₈NO₅ 210,0402; trouvé 210,0404.

### 2-Bromo-1-(4-hydroxy-3-méthoxy-5-nitrophényl)éthanone (3) (J. Borgulya, H. Bruderer, K. Bernauer, G. Zürcher, M. Da Prada, Helv. Chim. Acta, 1989, 72, 952-968).

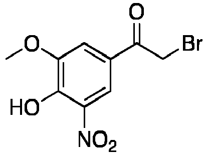

A une solution de 1-(4-hydroxy-3-méthoxy-5-nitrophényl)éthanone (9,6 g; 45,5 mmol) dans CH₂Cl₂ (200 mL) à TA, on ajoute graduellement du brome (2,4 mL; 46,8 mmol) et le mélange réactionnel est agité pendant 10 min. Durant cette période la couleur du brome disparaît totalement. Du pentane (400 mL) est alors ajouté. Après 10 min les cristaux jaunes sont filtrés sous une hotte, lavés avec un mélange pentane/éther 1:1, et séchés pour donner 3 (10 g; 76%), pf 143-145 °C (lit. 147-149 °C; J. Borgulya, H. Bruderer, K. Bernauer, G. Zürcher, M. Da Prada, Helv. Chim. Acta, 1989, 72, 952-968). RMN ¹H (CDCl₃; 300 MHz) *δ* (ppm) 11,18 (1 H; s), 8,36 (1 H; d; *J=* 1,8 Hz); 7,77 (1 H; d; *J=* 1,8 Hz); 4,44 (2 H; s); 4.03 (3 H; s). RMN ¹³C (CDCl₃; 75 MHz) *δ* (ppm) 188,7; 150,7; 150,6; 133,0; 125,0; 118,3; 115,9; 57,0; 29,4. IR (neat) *v*ₘₐₓ cm⁻¹ 3366; 3093; 2991; 2942; 1765; 1689; 1608; 1535; 1386; 1246; 1151; 1060; 912; 887; 850; 762. SMHR (ES) (*m*/*z*) [M-H]⁻ calculé pour C₉H₇⁷⁹BrNO₅ 287,9508; trouvé 287,9480.

### Préparation du 2-(4-hydroxy-3-méthoxy-5-nitrophényl)acétaldéhyde (4)

### 4-Allyl-2-méthoxy-6-nitrophénol (D. E. Lewin, A. Lowy, J. Am. Chem. Soc., 1933, 55, 1995-2000).

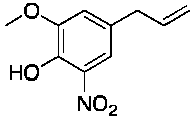

A une solution d'eugénol (6,76 g; 41,2 mmol) dans l'acide acétique (100 mL) à TA, on ajoute HNO₃ à 70% (2,8 mL; 44,2 mmol). Le mélange réactionnel est agité pendant 15 min, extrait avec de l'éther, lavé vigoureusement avec de la saumure, et finalement avec un tampon phosphate (pH = 7). La solution éthérée est diluée avec le même volume de pentane, filtrée sur gel de silice et évaporée sous pression réduite pour donner le 4-allyl-2-méthoxy-6-nitrophénol (6,36 g, 79%) sous forme d'huile rouge foncé. RMN ¹H (CDCl₃; 300 MHz) *δ* (ppm) 10,61 (1 H; sl), 7,48 (1 H; d; *J=* 1,5 Hz); 6,96 (1 H; d; *J* = 1,5 Hz); 5,91 (1 H; m); 5,20-5,08 (2 H; m); 3,92 (3 H; s); 3,35 (2 H; d; *J =* 6,6 Hz). RMN ¹³C (CDCl₃; 75 MHz) *δ* (ppm) 149,8; 144,8; 135,9; 133,6; 131,2; 118,6; 117,1; 115,0; 56,7; 39,4. IR (neat) *v*ₘₐₓ cm⁻¹ 3225; 3080; 2976; 2939; 1639; 1539; 1428; 1390; 1326; 1259; 1133; 1061; 916; 763. SMHR (ES) (*m*/*z*) [M+Na]⁺ calculé pour C₁₀H₁₁NNaO₄ 232,0586; trouvé 232,0578.

### 2-(4-Hydroxy-3-méthoxy-5-nitrophényl)acétaldéhyde (4)

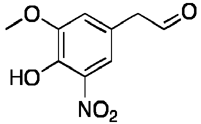

A une solution de 4-allyl-2-méthoxy-6-nitrophénol (2,0 g; 9,6 mmol) dans du THF (23 mL) et de l'eau (8 mL) à 0 °C, on ajoute une solution de tétroxyde d'osmium 0.166 M (23 mg; 0,09 mmol) dans un mélange THF/eau 7:5 (0,55 mL). Après 5 min, on ajoute NaIO₄ (4,8 g; 22 mmol). Le mélange réactionnel est agité pendant 1 h à 0 °C, laissé revenir graduellement à TA pendant 1 h, extrait avec un mélange CH₂Cl₂/éther 1:1 et la phase organique est lavée une fois avec de la saumure, séchée sur Na₂SO₄, et évaporée sous pression réduite à 25 °C. Le résidu est dissous dans le volume minimal de CH₂Cl₂ et un mélange pentane/éther 1:1 est ajouté. Le solvant est évaporé sous pression réduite à 25 °C jusqu'à formation de cristaux jaunes; du pentane est ajouté et la solution est concentrée encore une fois. Les cristaux sont filtrés pour donner 4 (1.66 g, 82%), pf 130-132 °C. RNLN ¹H (CDCl₃; 300 MHz) *δ* (ppm) 10,71 (1 H; s), 9,80 (1 H; t; *J* = 1,8 Hz); 7,57 (1 H; d; *J* = 1,8 Hz), 6,97 (1 H; d; *J* = 1,8 Hz), 3,96 (3 H; s); 3,74 (2 H; d; *J* = 1,8 Hz). RMN ¹³C (CDCl₃; 75 MHz) *δ* (ppm) 197,8; 150,4; 145,8; 133,8; 123,1; 119,0; 116,6; 56,8; 49,5. IR (neat) *v*ₘₐₓ cm⁻¹ 3226; 1720; 1583; 1449; 1333; 1264; 1238; 1134; 1064; 921; 764. SMHR (ES) (*m*/*z*) [M-H]⁻ calculé pour C₉H₈NO₅ 210,0402; trouvé 210,0399.

### 5-(1-(3-(Azacyclotridécan-1-yl)propyl)-4-(4-hydroxy-3-méthoxy-5-nitrophényl)-1H-pyrrol-3-yl)-3-nitrobenzène-1,2-diol (5)

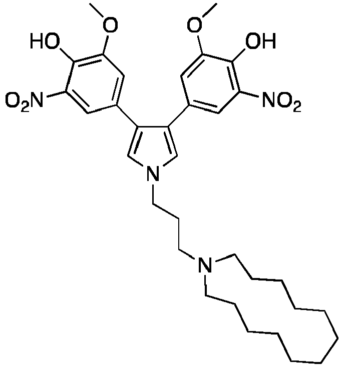

Le bromure de phénacyle 3 (1,23 g; 4,2 mmol) et NaI (3,2 g; 21 mmol) sont ajoutés à une solution de l'amine *2* (1 g, 4,2 mmol) dans le DMSO (20 mL) à TA sous argon et le mélange réactionnel est agité pendant 15 min à TA. Une solution de l'aldéhyde 4 (900 mg, 4,3 mmol) dans le méthanol (60 mL) est ensuite ajoutée et le mélange réactionnel est agité pendant 16 h à TA. Il est extrait avec CH₂Cl₂ et la phase organique est lavée avec une solution saturée de NaHCO₃, de la saumure, séchée sur Na₂SO₄, évaporée sous pression réduite et chromatographiée sur gel de silice (gradient d'élution de CH₂Cl₂ à CH₂Cl₂/MeOH 80:1) pour donner 5 (682 mg, 26%) sous forme d'un solide rouge amorphe. RMN ¹H (CDCl₃; 500 MHz) *δ* (ppm) 7,65 (2 H; d; *J =* 2,0 Hz); 7,01 (2 H; d; *J =* 2,0 Hz); 6,87 (2 H; s); 4,05 (2 H; t; *J =* 7,0 Hz); 3,77 (6 H; s); 2,70-2,43 (6 H; m); 2,15 (2 H; quint; *J* = 7,5 Hz); 1,66-1,34 (20 H; m). RMN ¹³C (CDCl₃; 75 MHz) *δ* (ppm) 149,7; 144,8; 133,9; 126,7; 121,0; 120,7; 118,7; 114,5; 56,8; 53,0; 51,7; 47,7; 27,9; 25,7; 25,6; 25,5; 25,2; 23,9. IR (neat) *v*ₘₐₓ cm⁻¹ 3255; 2930; 2859; 1552; 1522; 1464; 1337; 1240; 1156; 919; 732. SMHR (ES) (*m*/*z*) [M+H]⁺ calculé pour C₃₃H₄₅N₄O₈ 625,3237; trouvé 625,3228.

### 5,5'-(1-(3-(Azacyclotridécan-1-yl)propyl)-1H-pyrrole-3,4-diyl)bis(3-nitrobenzène-1,2-diol) (1)

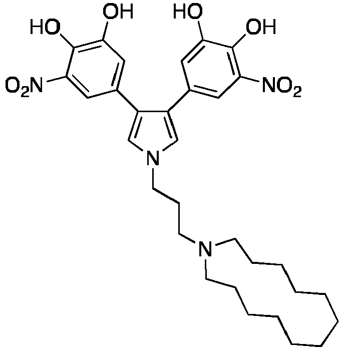

Au composé 5 (50 mg; 0,080 mmol) sous argon à TA, on ajoute BBr₃ (solution 1 M dans CH₂Cl₂, 2 mL; 2 mmol). Le mélange hétérogène est agité pendant 30 min à TA. On ajoute un mélange CH₂Cl₂/pentane 1:1 (30 mL) et, après 5 min, le précipité est séparé, lavé avec le même mélange de solvants et dissous dans le volume minimal de CH₂Cl₂/éthanol 10:1. La solution est introduite sur une colonne de gel de silice, éluée avec CH₂Cl₂/éthanol 20:1 et évaporée sous pression réduite pour donner 1 sous forme de solide amorphe rouge-orange (44 mg; 92%). RMN ¹H (CD₃OD; 300 MHz) *δ* (ppm) 7,44 (2 H; d; *J=* 1,8 Hz); 7,00 (2 H; s); 6,97 (2 H; d; *J=* 1,8 Hz); 4,12 (2 H; t; *J* = 6,3 Hz); 3,30-3,12 (6 H; m); 2,32 (2 H; quint; *J* = 6,0 Hz); 1,85-1,70 (4 H; m); 1,60-1,39 (16 H; m). RMN ¹³C (CD₃OD, 75 MHz) *δ* (ppm) 147,5; 141,9; 134,7; 127,0; 121,4; 121,3; 120,5; 113,5; 52,4; 52,0; 46,1; 25,9; 25,3; 24,6; 24,2; 23,9; 21,2. IR (neat) *v*ₘₐₓ cm⁻¹ 3379; 2932; 1621; 1555; 1471; 1300; 1236; 803; 762; 667. SMHR (ES) (*m*/*z*) [M+H]⁺ calculé pour C₃₁H₄₁N₄O₈ 597,2924; trouvé 597,2938.

### Chlorhydrate 1●HCl

A une solution de 1 dans un volume minimal de dichlorométhane, on ajoute une solution de HCl 2 M dans le méthanol (5 équiv) diluée dans de l'éther. Le précipité formé est alors filtré et séché. Pf 179-184 °C (déc). RMN ¹H (DMSO-*d*₆; 300 MHz) *δ* (ppm) 7,19 (2 H; d; *J* = 3,0 Hz); 7,06 (2 H; s); 6,90 (2 H; d; *J* = 3,0 Hz); 4,00 (2 H; t; *J* = 6,0 Hz); 3,15-2,97 (6 H; m); 2,24 (2 H; quint; *J* = 6,0 Hz); 1,75-1,58 (4 H; m); 1,44-1,28 (16 H; m). RMN ¹³C (DMSO-*d*₆; 75 MHz) *δ* (ppm) 148,0; 140,4; 137,7; 126,7; 121,3; 120,5; 120,1; 113,5; 51,9; 51,2; 46,7; 25,9; 25,7; 24,9; 24,7; 24,4; 20,9. IR (neat) *v*ₘₐₓ cm⁻¹ 3111; 2930; 2861; 1552; 1463; 1293; 1233; 1061; 866; 801; 762. SMHR (ES) (*m*/*z*) [M-HCl-H]⁻ calculé pour C₃₁H₃₉N₄O₈ 595,2768; trouvé 595,2773.

### Etude biologique in vitro

L'activité biologique du composé (*1)* a été étudiée *in vitro* sur 3 lignées cellulaires cancéreuses différentes:
- HCT-116 (carcinome colorectal)
- U87 (glioblastome)
- K562 (leucémie myéloïde)
ainsi que sur 3 types cellulaires normaux non cancéreux :
- HUVEC (cellules endothéliales ombilicales vasculaires humaines).
- NHDF (fibroblastes de derme humain)
- HFDPC (cellules papillaires dermiques dérivées de follicules de cheveux humains).

Les cellules sélectionnées pour cette étude ont été incubées à 37°C en présence du composé *(1)* ajouté dans le milieu de culture à différentes concentrations et à différents temps.

L'ensemble des expériences réalisées a permis de déterminer le degré de toxicité du composé testé, d'étudier son effet sur le déroulement du cycle cellulaire ainsi que sa capacité à induire une réponse autophagique et une mort cellulaire par apoptose. Nous avons étudié son mode d'action afin de déterminer sa cible moléculaire.

### 1. Etude de l'effet du composé (1) sur la croissance cellulaire

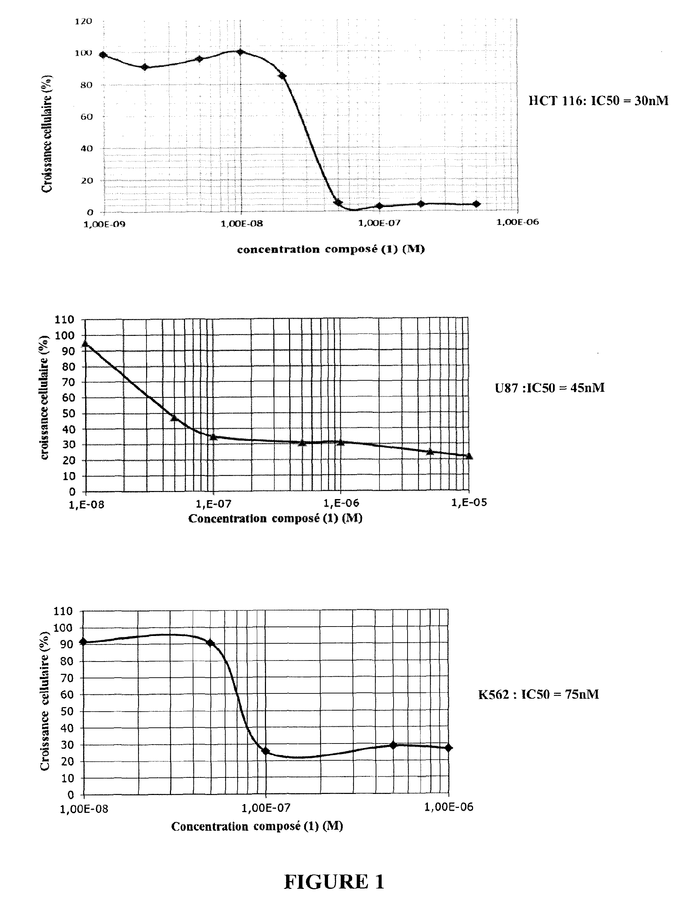

L'effet du composé *(1)* sur la croissance des cellules HCT-116, U87 et K562 a été évalué l'aide d'un test de croissance cellulaire (CellTiter-Blue™ Cell Viability Assay, Promega). Les courbes de croissance ainsi que les valeurs des IC50 (concentration du composé qui induit la diminution de la croissance cellulaire de 50%) déterminées après 72h du traitement des cellules avec le composé *(1)* sont présentées dans la Figure 1. Elles sont de 30 nM pour les cellules HCT-116, 45 nM pour les cellules U87 et 70 nM pour les K562.

L'effet du composé *(1)* sur la croissance cellulaire a été décrit de façon plus détaillée sur la lignée cellulaire HCT-116. Cette étude a été réalisée en fonction de la concentration et du temps d'exposition des cellules à la molécule testée (24h, 48h et 72h) par le comptage du nombre de cellules vivantes après exclusion des cellules mortes par coloration au bleu de trypan.

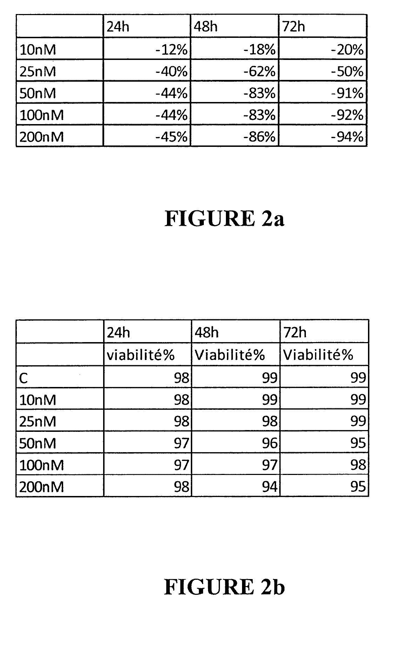

La Figure 2 représente l'effet du composé *(I)* sur le nombre et la viabilité des cellules HCT 116 en fonction de sa concentration et du temps de traitement.

Les résultats présentés dans la Figure 2a montrent que le nombre de cellules diminue en fonction de la concentration et du temps de traitement avec le composé *(1)*. Le composé *(1)* à la concentration de 25 nM manifeste déjà une activité dès 24 heures de traitement en réduisant le nombre de cellules de 40%, allant jusqu'à plus de 80% d'inhibition dès 48 heures pour les concentrations supérieures à l'IC50. Cet effet inhibiteur est cependant associé à une absence de modification de la viabilité cellulaire (Figure 2b). Ceci indique que l'action du composé *(1)* est cytostatique et non cytotoxique.

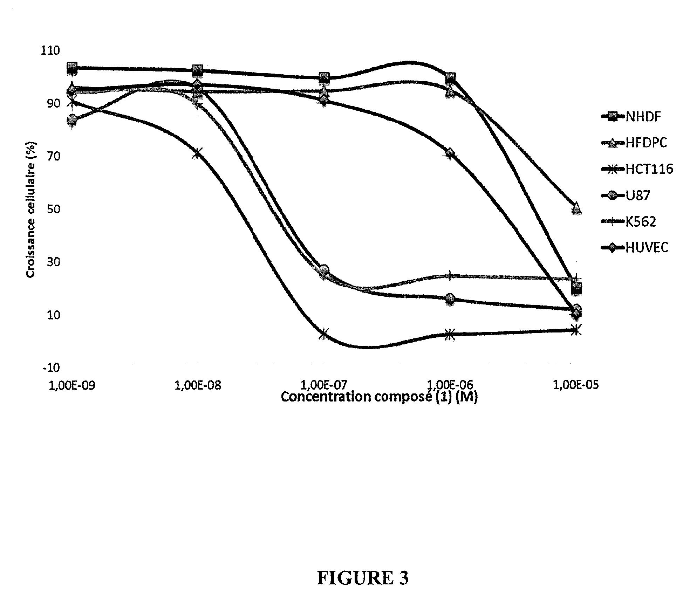

Afin de déterminer la spécificité d'action du composé *(1)* sur les cellules cancéreuses, nous avons évalué son effet vis-à-vis de cellules normales humaines HUVEC, NHDF et HFDPC et des légendes de cellule cancéreuse HCT-116, U87 et K652. Les résultats présentés dans la Figure 3 montrent que l'IC50 déterminé après 72h de traitement avec le composé *(1)* est 2 µM pour les cellules HUVEC, 5 µM pour les cellules NHDF et 10 µM pour les HFDPC.

Les valeurs des IC50 sont ainsi, jusqu'à 300 fois plus élevées pour les cellules normales par rapport à celles qui sont obtenues pour les cellules cancéreuses. Ceci indique clairement que les cellules malignes sont significativement plus sensibles à l'action du composé *(1)* par rapport aux cellules saines.

### 2. Etude de l'effet du composé (1) sur le cycle cellulaire

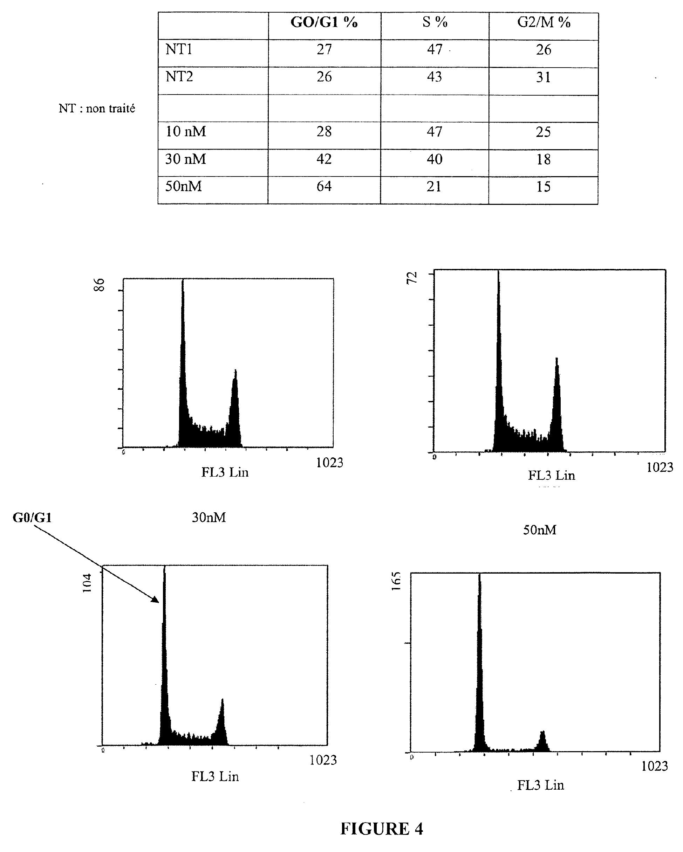

L'analyse par cytométrie en flux (FC500, Beckman Coulter) des cellules HCT-116 traitées avec le composé *(1)* a montré que ce composé bloque la division cellulaire en phase G0/G1. Cet effet est significatif après 24 heures d'exposition des cellules au composé *(1)* utilisé aux concentrations de 30 et 50 nM (Figure 4).

### 3. Etude de l'effet du composé (1) sur l'expression des protéines p21 et p27 impliquées dans l'arrêt du cycle cellulaire

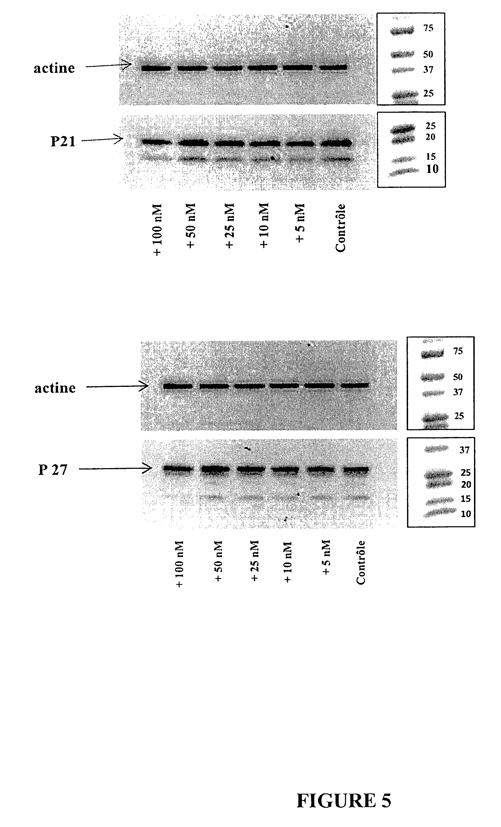

À partir des résultats précédents montrant un arrêt du cycle en G0/G1, associé à une diminution du nombre de cellules en l'absence de toxicité, nous avons évalué l'expression des protéines p21 et p27 associées à l'arrêt du cycle cellulaire en G0/G1. Le gène de la protéine p27 est décrit comme étant régulé par FOXO3, alors que celui de la protéine p21 est contrôlé par le facteur de transcription p53, stimulé entre autre par le stress oxydatif. Les cellules HCT 116 ont été traitées par le composé *(1)* pendant 24h. Les extraits cellulaires ont été ensuite analysés par Western blot. Les résultats présentés dans la Figure 5 montrent une augmentation de l'expression de la protéine p27 suite au traitement avec le composé *(1)* aux concentrations 25 et 50 nM. Le composé *(1)* n'a aucun effet significatif sur l'expression de la protéine p21.

### 4. Etude de l'effet du composé (1) sur l'induction de l'apoptose

Afin d'affirmer que le composé *(1)* n'induit pas la mort cellulaire par apoptose, les cellules HCT-116 traitées pendant 24h ont été analysées pour évaluer l'activité des caspases 3 et 7 et la modification du potentiel transmembranaire mitochondrial.

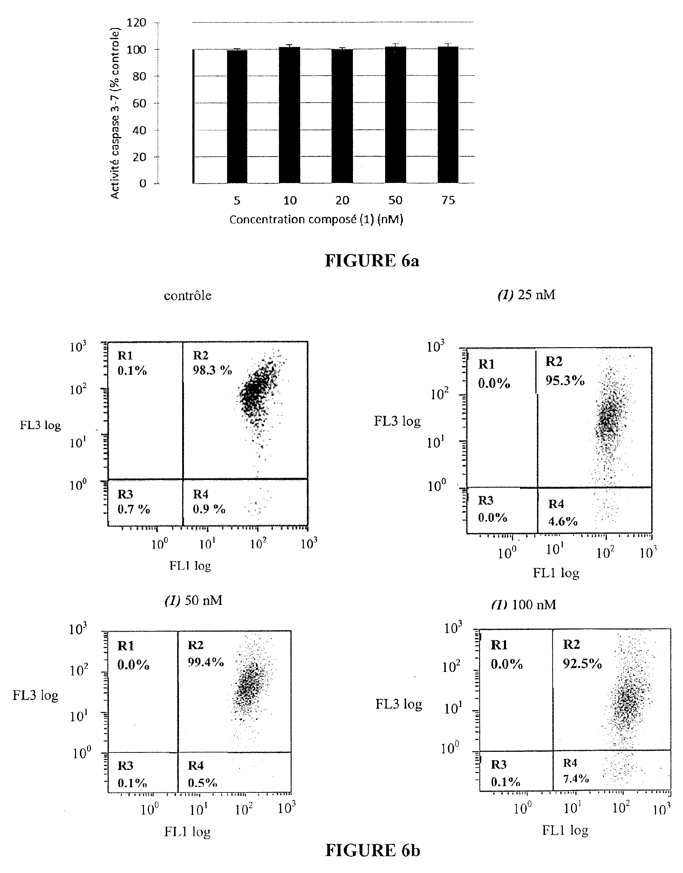

L'activité des caspases 3 et 7, enzymes marqueurs de l'apoptose, a été mesurée à l'aide du test Apo-ONE (Promega) après traitement des cellules HCT-116 avec le composé *(1)* aux concentrations variantes de 5 nM à 75 nM. Les résultats présentés dans la Figure 6a montrent l'absence de variations dans l'activité de ces enzymes suite au traitement avec la molécule testée.

L'étude du potentiel transmembranaire mitochondrial, Δψm, a été réalisée par cytométrie en flux, en utilisant la sonde JC-1 (MitoProbe™ JC-1 assay, Molecular Probes). JC-1 est un composé anionique lipophile qui pénètre sélectivement dans la mitochondrie, dont la couleur change du vert au rouge lorsque le potentiel transmembranaire augmente. Contrairement aux cellules saines, les cellules en apoptose, présentent un faible Δψm qui est associé à une forte coloration verte de la sonde JC-1.

Les résultats présentés dans la Figure 6b montrent que le traitement des cellules HCT-116 avec le composé *(1)* aux concentrations variantes de 25 nM à 100 nM ne modifie pas significativement le Δψm. L'ensemble de ces résultats indique que le composé *(1)* n'induit pas la voie apoptotique dépendante des caspases (mort cellulaire programmé de type I).

### 5. Etude de l'autophagie

Dans le but de savoir si l'arrêt du cycle cellulaire induit par le composé *(1)* entraîne une réaction autophagique, les cellules HCT-116 ont été traitées par le composé *(1)* à différentes doses pendant 24 heures. Les extraits cellulaires ont été ensuite soumis à l'analyse par Western blot afin de visualiser l'expression de la protéine LC3-II. En effet, la protéine LC3 existe dans la cellule soit sous forme cytosolique (LC3-I) soit associée aux phagosomes (LC3-II). Cette dernière est le marqueur de la réaction autophagique.

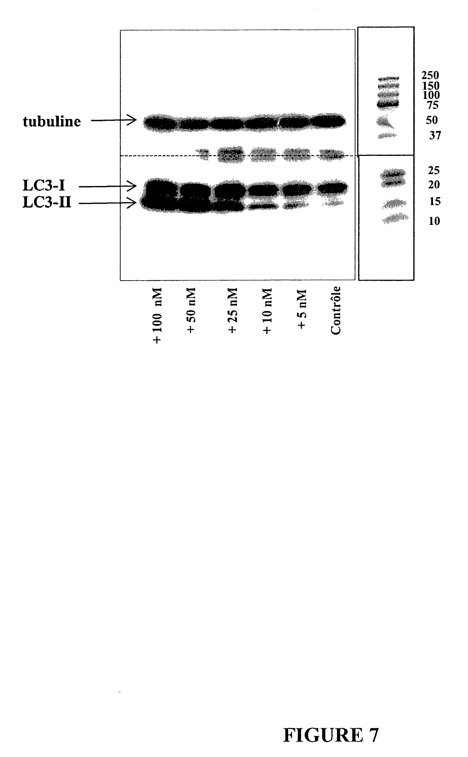

Les résultats présentés dans la Figure 7 montrent clairement une augmentation de l'expression de LC3-II en présence du composé *(1)* utilisé aux concentrations variants de 5 nM à 100 nM.

### 6. Etude du mode d'action du composé (1)

Compte tenu de l'arrêt du cycle cellulaire en phase G0/G1 associé à une réponse autophagique en absence d'apoptose suite au traitement avec le composé *(1)*, nous avons étudié l'action de ce composé sur la voie de survie impliquant PI3 kinase - Akt - Foxo. Cette voie de signalisation se trouve très souvent dérégulée (par stimulation) dans un grand nombre de cellules cancéreuses et contribue à leur survie.

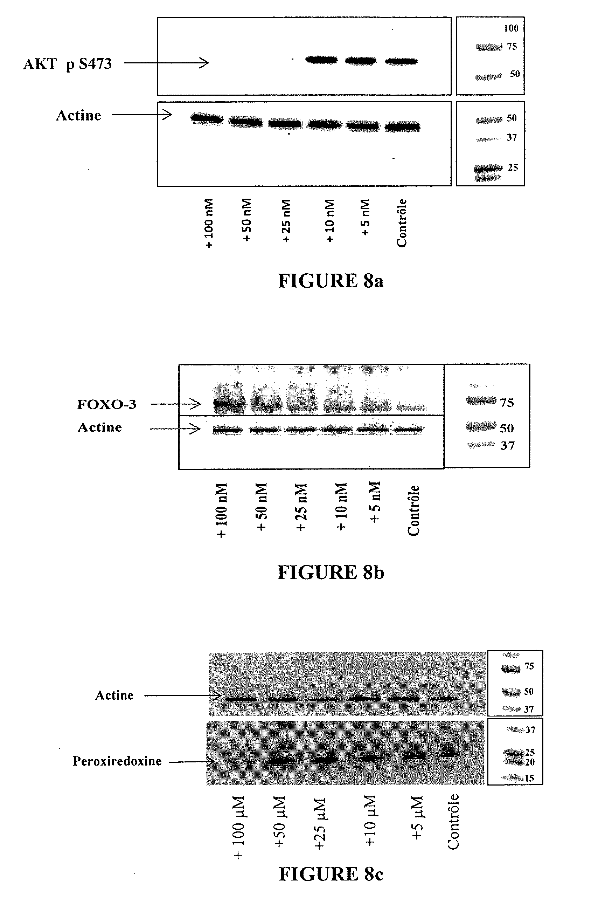

Les résultats présentés dans la Figure 8 montrent que le composé *(1)* bloque cette voie de survie. En effet, les cellules HCT-116 ont été exposées pendant 24h à l'action du composé *(1)* aux concentrations variant de 5 nM à 100 nM. L'analyse des extraits cellulaires par Western blot a révélé que le composé *(1)* dès la concentration de 25 nM supprime totalement la phosphorylation de la sérine 473 de la protéine Akt (Figure 8a). Il stimule l'expression de la protéine FOXO3 (Figure 8b), dont la synthèse est réduite par la protéine Akt-phosphorylée. Il stimule l'expression de la protéine peroxiredoxine 1, dont le gène est décrit comme étant sous contrôle du facteur FOXO3 (Figure 8c).

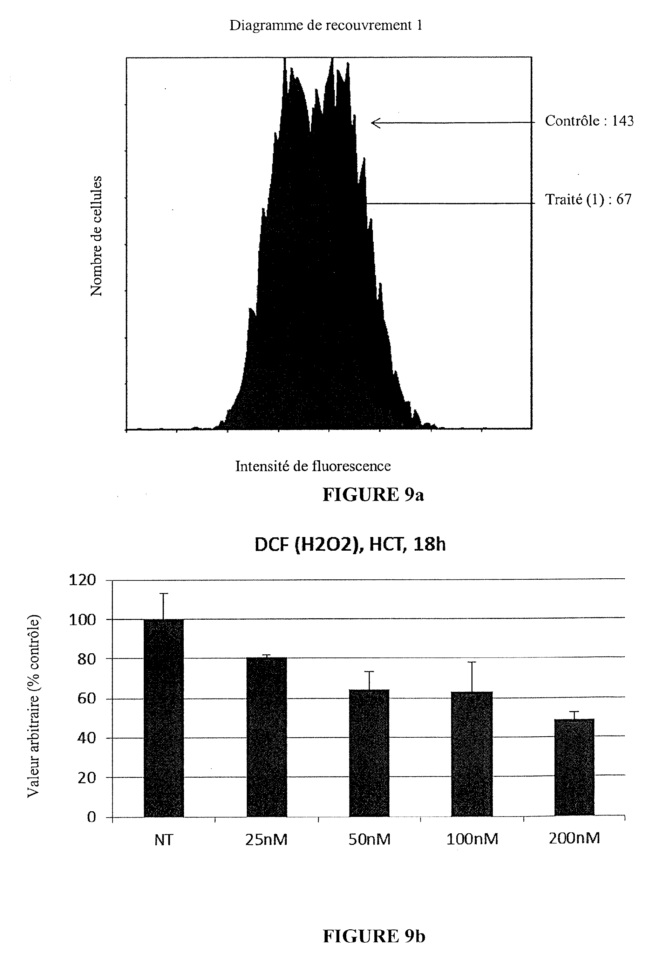

La protéine peroxiredoxine est une enzyme ayant une activité peroxydase, contrôlant la concentration de peroxyde d'hydrogène (H₂O₂) intracellulaire. Nous avons donc évalué l'effet du composé *(1)* sur la concentration d'H₂O₂ intracellulaire, en utilisant le DCF-DA (2',7'-dichlorofluorescein diacetate, Sigma) comme marqueur de l'H₂O₂ intracellulaire. Le peroxyde d'hydrogène est un second messager intracellulaire. Il régule, selon sa concentration, de nombreuses fonctions comme la prolifération ou l'apoptose. Une réduction de sa concentration est associée à une diminution de prolifération. Les résultats d'analyse en cytométrie en flux des cellules HCT-116 traitées par le composé *(1)* à la concentration de 50nM montrent une diminution d'environ 50% de la concentration d'H₂O₂ intracellulaire après 18 heures de traitement (Figure 9a). L'effet observé est dose dépendant (Figure 9b).

## Revendications

1. Composé de formule (I) suivante : 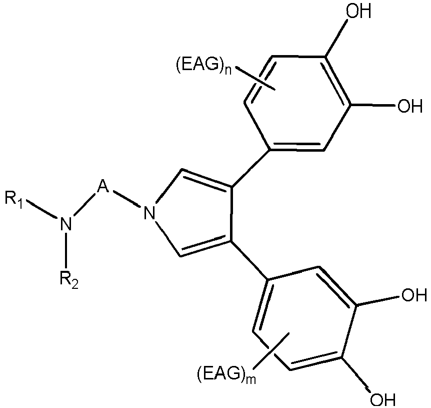 dans laquelle :
- m est un entier de 0 à 3, de préférence de 0 à 2 ;
- n est un entier de 0 à 3, de préférence de 0 à 2;
- m + n ≥ 1
- EAG représente un groupe électroattracteur indépendamment choisi parmi un atome d'halogène, un groupe NO₂, CF₃, CCl₃, CN, CO₂H, (C=O)NR₂, CH=NR, (C=S)OR, (C=O)SR, CS₂R, SO₂R, SO₂NR₂, SO₃R, P(O)(OR)₂, P(O)(R)₂, B(OR)₂, où R est un radical (C₁ - C₆) alkyle, un groupe phényle ou un atome d'hydrogène;
- A représente une chaîne hydrocarbonée, saturée ou insaturée, linéaire ou ramifiée, comprenant de 1 à 10 atomes de carbone ; et
- R₁ et R₂ représentent chacun, indépendamment l'un de l'autre, un atome d'hydrogène, un groupe CO-(C₁ - C₆)-alkyle, (C₁ - C₆) alkyle, phényle ou phényle-(C₁ - C₆)-alkyle, ou R₁ et R₂ forment ensemble, avec l'atome d'azote qui les porte, un hétérocycle à 5 à 15 chaînons, éventuellement substitué par un groupe (C₁ - C₆) alkyle ;
y compris ses stéréoisomères et leurs mélanges, ou un sel d'acide pharmaceutiquement acceptable de celui-ci.

2. Composé selon la revendication 1, **caractérisé en ce qu'**il s'agit d'un composé de formule (Ia) suivante : 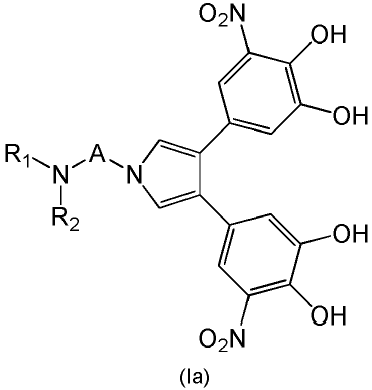 dans laquelle les radicaux A,
R₁ et R₂ ont les significations données à la revendication 1,
y compris ses stéréoisomères et leurs mélanges, ou un sel d'acide pharmaceutiquement acceptable de celui-ci.

3. Composé selon la revendication 1 ou 2, **caractérisé en ce que** R₁ et R₂ forment ensemble, avec l'atome d'azote qui les porte, un hétérocycle qui est saturé et qui ne comprend pas d'autre hétéroatome que l'atome d'azote qui porte R₁ et R₂.

4. Composé selon l'une quelconque des revendications 1 à 3, **caractérisé en ce que** R₁ et R₂ forment ensemble, avec l'atome d'azote qui les porte, un hétérocycle de 10 à 15 chaînons, de préférence à 13 chaînons.

5. Composé selon l'une quelconque des revendications 1 à 4, **caractérisé en ce que** A est une chaîne hydrocarbonée linéaire saturée comprenant 3 à 6 atomes de carbone, de préférence 3 atomes de carbone.

6. Composé selon l'une quelconque des revendications 1 à 5, correspondant au composé de formule (1) suivante : 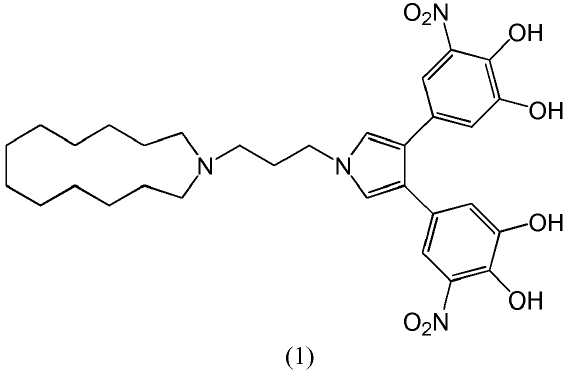 ou un sel d'acide pharmaceutiquement acceptable de celui-ci.

7. Composé selon l'une quelconque des revendications 1 à 6, **caractérisé en ce que** le sel d'acide pharmaceutiquement acceptable est un chlorhydrate.

8. Composition pharmaceutique comprenant au moins un composé selon l'une quelconque des revendications 1 à 7 et au moins un excipient pharmaceutiquement acceptable.

9. Composé selon l'une quelconque des revendications 1 à 7, pour son utilisation en tant que médicament.

10. Composé selon la revendication 9, pour son utilisation en tant que médicament pour le traitement du cancer.

11. Composé pour son utilisation selon la revendication 10, **caractérisé en ce que** le cancer est choisi parmi le cancer du poumon, du sein, du foie, de l'estomac, du côlon, de l'anus, de l'oesophage, du larynx, du nasopharynx, du pancréas, de la prostate, du rein, de la vessie, du duodénum, de l'endomètre, de la plèvre, de la peau, du testicule, de l'ovaire, de l'utérus, du cerveau, des os, de la bouche, de l'oeil, ou les cancers hématopoïétiques tels que les leucémies, les leucémies myéloïdes, les lymphomes, le cancer de la moelle osseuse ou encore les tumeurs d'origine neuroectodermique tels que les glioblastomes.

12. Procédé de préparation d'un composé de formule (I) selon l'une quelconque des revendications 1 à 7, comprenant :
a) la réaction entre une amine de formule (II) suivante, un halogénure de formule (III) suivante, et un aldéhyde de formule (IV) suivante, 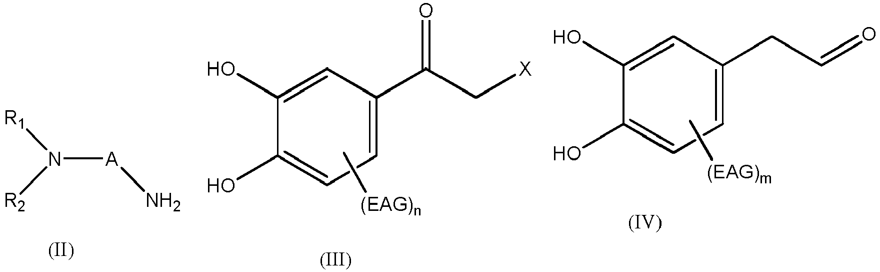 dans lesquelles n et m, EAG, R₁, R₂ et A sont tels que définis à la revendication 1 et X représente un atome d'halogène, pour donner un composé de formule (I) selon la revendication 1, et
b) éventuellement la formation d'un sel pharmaceutiquement acceptable à partir du composé de formule (I) obtenu à l'étape a) précédente.

13. Procédé de préparation d'un composé de formule (I) selon la revendication 12, **caractérisé en ce que** l'halogénure (III) et l'amine (II) sont d'abord mis à réagir ensemble avant d'ajouter l'aldéhyde (IV).

14. Procédé de préparation d'un composé de formule (I) selon l'une des revendications 12 et 13, **caractérisé en ce que** X est un atome de chlore, de brome ou d'iode.

## Patentansprüche

1. Verbindung der folgenden Formel (I): 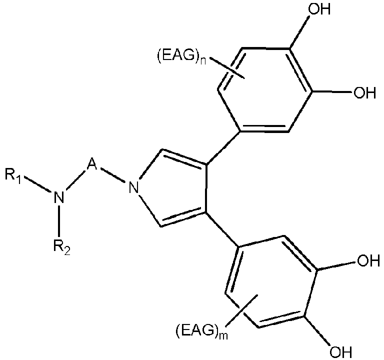 wobei:
- m eine ganze Zahl von 0 bis 3, vorzugsweise von 0 bis 2 ist;
- n eine ganze Zahl von 0 bis 3, vorzugsweise von 0 bis 2 ist;
- m + n ≥ 1
- EAG für eine elektronenziehende Gruppe steht, unabhängig ausgewählt aus einem Halogenatom, einer Gruppe NO₂, CF₃, CCl₃, CN, CO₂H, (C=O)NR₂, CH=NR, (C=S)OR, (C=O)SR, CS₂R, SO₂R, SO₂NR₂, SO₃R, P(O)(OR)₂, P(O)(R)₂, B(OR)₂, wobei R ein (C₁ - C₆) -Alkylrest, eine Phenylgruppe oder ein Wasserstoffatom ist;
- A für eine gesättigte oder ungesättigte, geradkettige oder verzweigte Kohlenwasserstoffkette steht, die 1 bis 10 Kohlenstoffatome umfasst; und
- Rᵢ und R₂, unabhängig voneinander, jeweils für ein Wasserstoffatom, eine CO- (C₁-C₆) -Alkyl-, (C₁-C₆)-Alkyl-, Phenyl- oder Phenyl-(C₁-C₆)-Alkylgruppe stehen, oder Rᵢ und R₂ zusammen, mit dem sie tragenden Stickstoffatom, einen 5- bis 15-gliedrigen Heterocyclus bilden, gegebenenfalls durch eine (C₁-C₆)-Alkylgruppe substituiert;
einschließlich ihrer Stereoisomere und deren Mischungen, oder eines pharmazeutisch annehmbaren Säuresalzes hievon.

2. Verbindung nach Anspruch 1, **dadurch gekennzeichnet, dass** es sich um eine Verbindung der folgenden Formel (Ia) handelt: 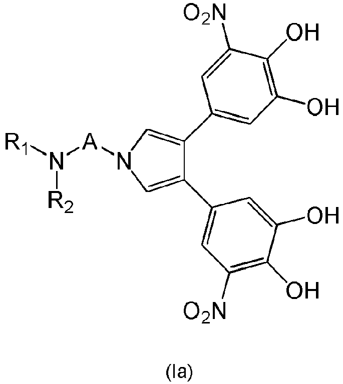 wobei die Reste A, R₁ und R₂ die in Anspruch 1 angegebenen Bedeutungen haben, einschließlich ihrer Stereoisomere und deren Mischungen, oder eines pharmazeutisch annehmbaren Säuresalzes hievon.

3. Verbindung nach Anspruch 1 oder 2, **dadurch gekennzeichnet, dass** R₁ und R₂ zusammen, mit dem sie tragenden Stickstoffatom, einen Heterocyclus bilden, der gesättigt ist und kein anderes Heteroatom als das R₁ und R₂ tragende Stickstoffatom umfasst.

4. Verbindung nach einem der Ansprüche 1 bis 3, **dadurch gekennzeichnet, dass** R₁ und R₂ zusammen, mit dem sie tragenden Stickstoffatom, einen 10- bis 15-gliedrigen, vorzugsweise 13-gliedrigen, Heterocyclus bilden.

5. Verbindung nach einem der Ansprüche 1 bis 4, **dadurch gekennzeichnet, dass** A eine gesättigte geradkettige Kohlenwasserstoffkette ist, die 3 bis 6 Kohlenstoffatome, vorzugsweise 3 Kohlenstoffatome, umfasst.

6. Verbindung nach einem der Ansprüche 1 bis 5, gemäß der Verbindung der folgenden Formel (1): 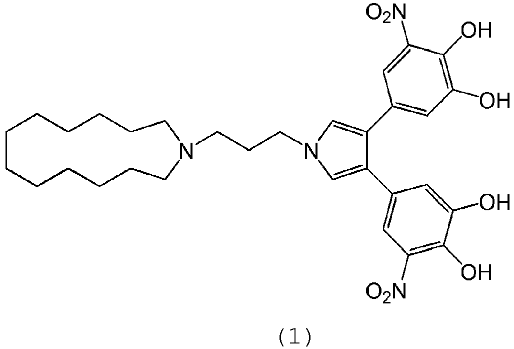 oder ein pharmazeutisch annehmbare Säuresalz hievon.

7. Verbindung nach einem der Ansprüche 1 bis 6, **dadurch gekennzeichnet, dass** das pharmazeutisch annehmbare Säuresalz ein Hydrochlorid ist.

8. Pharmazeutische Zusammensetzung, mindestens eine Verbindung nach einem der Ansprüche 1 bis 7 und mindestens einen pharmazeutisch annehmbaren Träger umfassend.

9. Verbindung nach einem der Ansprüche 1 bis 7, für deren Verwendung als Medikament.

10. Verbindung nach Anspruch 9, für deren Verwendung als Medikament für die Behandlung von Krebs.

11. Verbindung für deren Verwendung nach Anspruch 10, **dadurch gekennzeichnet, dass** der Krebs aus Lungen-, Brust-, Leber-, Magen-, Kolon-, Anus-, Speiseröhren-, Kehlkopf-, Nasenrachen-, Bauchspeicheldrüsen-, Prostata-, Nieren-, Blasen-, Zwölffingerdarm-, Endometrium-, Brustfell-, Haut-, Hoden-, Eierstock-, Gebärmutter-, Gehirn-, Knochen-, Mund-, Augenkrebs, oder aus hämatopoetischen Krebsarten wie Leukämien, myeloische Leukämien, Lymphome, Knochenmarkkrebs oder auch aus Tumoren neuroektodermalen Ursprungs wie Glioblastome ausgewählt ist.

12. Verfahren zur Herstellung einer Verbindung der Formel (I) nach einem der Ansprüche 1 bis 7, Folgendes umfassend:
a) Reaktion zwischen einem Amin der folgenden Formel (II), einem Halogenid der folgenden Formel (III) und einem Aldehyd der folgenden Formel (IV), 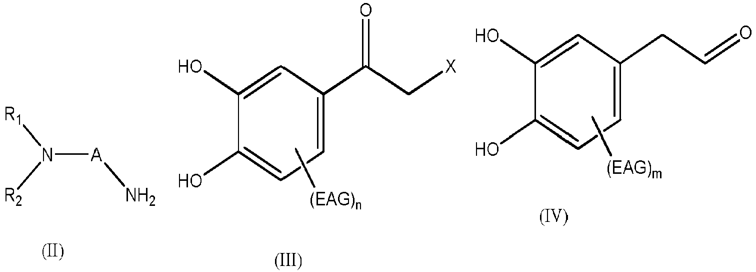 wobei n und m, EAG, R₁, R₂ und A wie in Anspruch 1 definiert sind und X für ein Halogenatom steht, um eine Verbindung der Formel (I) nach Anspruch 1 zu ergeben, und
b) gegebenenfalls die Bildung eines pharmazeutisch annehmbaren Salzes ausgehend von der Verbindung der Formel (I), wie im vorhergehenden Schritt a) erhalten.

13. Verfahren zur Herstellung einer Verbindung der Formel (I) nach Anspruch 12, **dadurch gekennzeichnet, dass** das Halogenid (III) und das Amin (II) zuerst miteinander umgesetzt werden, bevor die Zugabe des Aldehyds (IV) erfolgt.

14. Verfahren zur Herstellung einer Verbindung der Formel (I) nach einem der Ansprüche 12 und 13, **dadurch gekennzeichnet, dass** X ein Chlor-, Brom- oder Jodatom ist.

## Claims

1. Compound having the following formula (I): 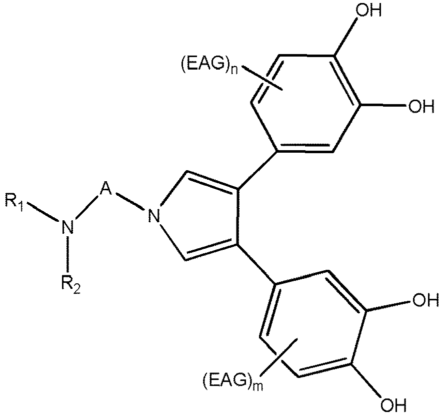 wherein:
- m is an integer from 0 to 3, preferably from 0 to 2;
- n is an integer from 0 to 3, preferably from 0 to 2;
- m + n ≥ 1
- EAG is an electro-attractive group chosen independently from among a halogen atom, an NO₂, CF₃, CCl₃, CN, CO₂H, (C=O)NR₂, CH=NR, (C=S) OR, (C=O)SR, CS₂R, SO₂R, SO₂NR₂, SO₃R, P(O)(OR)₂, P(O)(R)₂, B(OR)₂ group, where R is an alkyl (C₁-C₆) radical, a phenyl group or a hydrogen atom;
- A is a saturated or unsaturated, linear or branched hydrocarbon chain including 1 to 10 atoms of carbon; and
- R₁ and R₂ each represent, independently from one another, a hydrogen atom, a CO-(C₁-C₆)-alkyl, (C₁-C₆) alkyl, phenyl or phenyl-(C₁-C₆)-alkyl group, or R₁ and R₂ form, together with the nitrogen atom bearing same, a 5- to 15-member heterocycle, optionally substituted by a (C₁-C₆) alkyl group;
including the stereoisomers and the mixtures thereof, or a pharmaceutically acceptable acid salt thereof.

2. Compound according to claim 1, **characterised in that** it consists of a compound having the following formula (Ia): 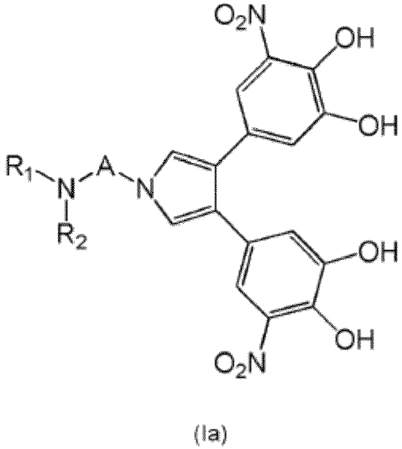 wherein the radicals A, R₁ and R₂ have the meanings given in claim 1, including the stereoisomers and the mixtures thereof, or a pharmaceutically acceptable acid salt thereof.

3. Compound according to claim 1 or 2, **characterised in that** the radicals R₁ and R₂ form, together with the nitrogen atom bearing same, a heterocycle which is saturated and does not comprise any other heteroatom than the nitrogen atom bearing R₁ and R₂.

4. Compound according to any of claims 1 to 3, **characterised in that** R₁ and R₂ form, together with the nitrogen atom bearing same, a 10- to 15-member, preferably 13-member, heterocycle.

5. Compound according to any of claims 1 to 4, **characterised in that** A is a saturated linear hydrocarbon chain comprising 3 to 6 carbon atoms, preferably 3 carbon atoms.

6. Compound according to any of claims 1 to 5, corresponding to the compound having the following formula (1): 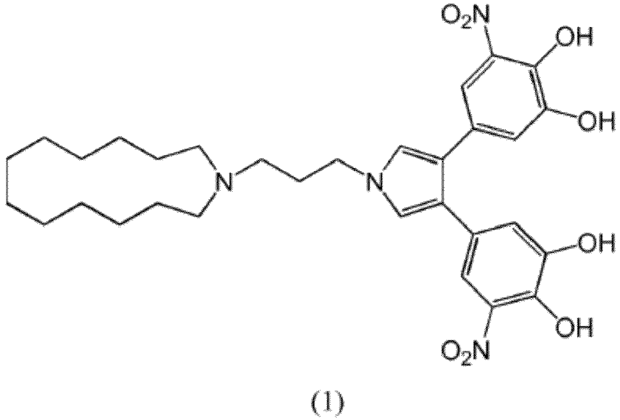 or a pharmaceutically acceptable acid salt thereof.

7. Compound according to any of claims 1 to 6, **characterised in that** the pharmaceutically acceptable acid salt is a hydrochloride.

8. Pharmaceutical composition comprising at least one compound according to any of claims 1 to 7 and at least one pharmaceutically acceptable excipient.

9. Compound according to any of claims 1 to 7, for the use thereof as a drug.

10. Compound according to claim 9, for the use thereof as a cancer treatment drug.

11. Compound for the use thereof according to claim 10, **characterised in that** the cancer is chosen from lung, breast, liver, stomach, colon, rectal, oesophageal, laryngeal, nasopharyngeal, pancreatic, prostate, kidney, bladder, duodenal, endometrial, pleural, skin, testicular, ovarian, uterine, brain, bone, oral, ocular cancer, or haematopoietic cancers such as leukaemias, myeloid leukaemias, lymphomas, bone marrow cancer or tumours of neuroectodermal origin such as glioblastomas.

12. Process for preparing a compound having formula (I) according to any of claims 1 to 7, comprising:
a) reaction between an amine having the following formula (II), a halide having the following formula (III), and an aldehyde having the following formula (IV), 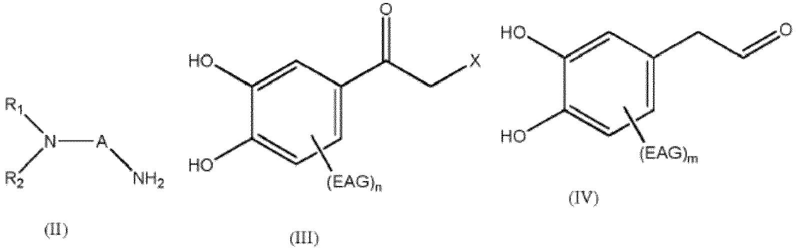 wherein n and m, EAG, R₁, R₂ and A are as defined in claim 1 and X represents a halogen atom, to arrive at a compound having formula (I) according to claim 1, and
b) optionally, formation of a pharmaceutically acceptable salt from the compound having formula (I) obtained in the previous step a).

13. Process for preparing a compound having formula (I) according to claim 12, **characterised in that** the halide (III) and the amine (II) are first allowed to react with one another before adding the aldehyde (IV).

14. Process for preparing a compound having formula (I) according to any of claims 12 and 13, **characterised in that** X is a chlorine, bromine or iodine atom.
